Europäisches Patentamt

European Patent Office

Office européen des brevets

(11) Numéro de publication: **0 258 080 B1**

# FASCICULE DE BREVET EUROPEEN

(45) Date de publication de fascicule du brevet:
**15.01.92**

(51) Int. Cl.⁵: **C23G 5/028**, C07C 17/42

(21) Numéro de dépôt: **87401628.0**

(22) Date de dépôt: **09.07.87**

Le dossier contient des informations techniques présentées postérieurement au dépôt de la demande et ne figurant pas dans le présent fascicule.

(54) **Composition à base de chlorure de méthylène - son utilisation pour le dégraissage des métaux.**

(30) Priorité: **21.07.86 FR 8610527**

(43) Date de publication de la demande:
**02.03.88 Bulletin 88/09**

(45) Mention de la délivrance du brevet:
**15.01.92 Bulletin 92/03**

(84) Etats contractants désignés:
**AT BE CH DE FR GB GR IT LI LU NL SE**

(56) Documents cités:
**FR-A- 2 029 261
US-A- 3 860 665
US-H- 334 985**

(73) Titulaire: **ATOCHEM
4 & 8, Cours Michelet La Défense 10
F-92800 Puteaux(FR)**

(72) Inventeur: **Jiskra, Alfred
9 Avenue Louise Bettignies
F-92700 Colombes(FR)**
Inventeur: **Perrot, Jacques
18 Rue de Franconville
F-95220 Herblay(FR)**

Rank Xerox (UK) Business Services

**Description**

La présente invention concerne une composition à base de chlorure de méthylène et son utilisation pour dégraisser les métaux.

Les hydrocarbures chlorés grâce à leur pouvoir solvant, leur ininflammabilité, leur point d'ébullition relativement bas sont le plus souvent employés pour le dégraissage des métaux, des textiles, des céramiques et des verres.

Le chlorure de méthylène est particulièrement stable à l'oxydation, l'hydrolyse, la pyrolyse. Il ne participe à aucune réaction photochimique. Son point d'ébullition bas permet son utilisation à faible température. Toutes ces propriétés en font donc un solvant de choix dans le dégraissage des métaux.

Cependant, lors de cette opération, il peut réagir avec des composés aromatiques introduits lors de l'usinage de métaux légers.

Selon un mécanisme réactionnel (type Friedel et Crafts) on a alors formation d'acide chlorhydrique, de composés plus lourds qui polluent le chlorure de méthylène. Il y a donc un risque d'explosion par décomposition (formation de chlorure d'acide, phosgène, gaz chlorhydrique).

Pour pallier ces inconvénients, il est nécessaire d'ajouter au chlorure de méthylène de petites quantités de produits organiques qui empêchent cette réaction : c'est la stabilisation.

On a déjà proposé des compositions de chlorure de méthylène stabilisé. Le brevet US 3,887,628 décrit du chlorure de méthylène contenant 0,05 à 2 % d'oxyde de butylène et d'oxyde de propylène. Le brevet US 3,923,912 décrit du chlorure de méthylène contenant 0,05 à 2 % d'acétone ou de méthyléthylcétone et éventuellement de l'oxyde de propylène. Le brevet US 4,108,910 décrit du chlorure de méthylène contenant un époxyde et un monoéther aliphatique. Le brevet européen EP 11 658 décrit du chlorure de méthylène stabilisé par du formiate d'éthyle ou de méthyle.

Le Brevet US-A- 3 860 665 décrit une composition contenant du chlorure de méthylène et de 0,05 % à 2 % en poids (par rapport au poids du chlorure de méthylène) de tétrahydrofurane et d'oxyde de propylène. Cependant, les compositions à deux stabilisants ne satisfont pas toujours aux différentes étapes du test BAM.

Un but de l'invention est donc de proposer une composition utile pour le dégraissage des métaux, et qui est stable en présence de métaux légers tels que l'aluminium ou leurs alliages.

La composition selon l'invention, à base de chlorure de méthylène, utile pour le dégraissage des métaux, comprend au moins un stabilisant. Elle se caractérise en ce qu'elle comprend, comme stabilisants :
(a) de l'oxyde de propylène ou de l'oxyde de butylène,
(b) de l'acétone, et
(c) du tétrahydrofurane
chacun des composés (a), (b) et (c) représentant entre 0,05 et 5 % en poids de la masse totale de la composition.

De préférence, la quantité de chacun des stabilisants (a), (b) et (c) représentent entre 0,2 et 2 % en poids de la masse totale de la composition.

La composition selon l'invention est préparée par simple mélange des produits avec le chlorure de méthylène. Ce mélange peut se faire à température ambiante et plus spécialement comprise entre 5 et 30°C.

La présente invention concerne aussi un procédé de dégraissage de métaux à l'aide de la composition selon l'invention. Le dégraissage peut se faire en phase vapeur ou en phase liquide selon des modes opératoires conventionnels.

EXEMPLE :

On prépare une composition de formule : (en % poids)

| - chlorure de méthylène | 98 |
| - oxyde de propylène | 0,5 |
| - acétone | 1 |
| - tétrahydrofuranne | 0,5 |

Cette composition réussit le passage du test BAM (Bundesanstalt für material prüfung, République Fédérale Allemande).

On rappelle que les 4 étapes du test BAM sont :

1) à 100 ml de composition on additionne 100 ml de toluène, 0,7 g de AlCl$_3$ 18 g de paillettes d'aluminium. Le mélange est maintenu à reflux pendant 18 h dans un bain marie à 58°C,

2) au mélange 1) on ajoute 1 g de stéarate de Zn puis reflux 18 h,

3) Au mélange 1) on ajoute 10 ml d'acide oleique puis reflux 18 h,

4) distillation de 300 ml de composition en 3 fractions de 100 ml puis test 1) sur chacune des 3 fractions.

Une formule peut être agréée par le BAM si elle ne présente aucune réaction au cours de ces essais.

**Revendications**

1. Composition à base de chlorure de méthylène, comprenant au moins un stabilisant, utile pour le dégraissage des métaux, caractérisée en ce qu'elle contient, comme stabilisants :
   a) de l'oxyde de propylène ou de l'oxyde de butylène,
   b) de l'acétone, et
   c) du tétrahydrofuranne
   chacun de ces composés (a), (b) et (c) représentant entre 0,05 et 5 % en poids de la masse totale de la composition.

2. Composition conforme à la revendication 1, caractérisée en ce que la quantité de chacun des composés (a), (b) et (c) est comprise entre 0,2 et 2 % en poids.

3. Composition conforme à l'une des revendications 1 et 2, caractérisée en ce qu'elle comprend comme stabilisants, de l'oxyde de propylène, de l'acétone et du tétrahydrofuranne.

4. Procédé de dégraissage de métaux, caractérisé en ce que lesdits métaux sont mis en contact avec une composition selon l'une des revendications 2 à 3.

**Claims**

1. Composition based on methylene chloride and comprising at least one stabiliser, useful for degreasing metals, characterised in that it contains, as stabilisers:
   a) propylene oxide or butylene oxide,
   b) acetone, and
   c) tetrahydrofuran,
   each of these compounds (a), (b) and (c) representing between 0.05 and 5% by weight of the total weight of the composition.

2. Composition according to Claim 1, characterised in that the amount of each of compounds (a), (b) and (c) is between 0.2 and 2% by weight.

3. Composition according to one of Claims 1 and 2, characterised in that it comprises, as stabilisers, propylene oxide, acetone and tetrahydrofuran.

4. Process for degreasing metals, characterised in that said metals are brought into contact with a composition according to one of Claims 2 and 3.

**Patentansprüche**

1. Zusammensetzung auf der Basis von Methylenchlorid, umfassend wenigstens einen Stabilisator zur Verwendung zum Entfetten von Metallen, dadurch gekennzeichnet, daß sie als Stabilisatoren enthält:
   a) Propylenoxid oder Butylenoxid
   b) Aceton und
   c) Tetradhydrofuran,
   wobei jede dieser Verbindungen (a), (b) und (c) zwischen 0,05 und 5 Gew.% der Gesamtmasse der Zusammensetzung ausmacht.

2. Zusammensetzung gemäß Anspruch 1, dadurch gekennzeichnet, daß die Menge jeder der Verbindungen (a), (b) und (c) zwischen 0,2 und 2 Gew.% beträgt.

**3.** Zusammensetzung gemäß einem der Ansprüche 1 und 2, dadurch gekennzeichnet, daß sie als Stabilisatoren Propylenoxid, Aceton und Tetrahydrofuran enthält.

**4.** Verfahren zum Entfetten von Metallen, dadurch gekennzeichnet, daß diese Metalle mit der Zusammensetzung gemäß einem der Ansprüche 2 und 3 in Kontakt gebracht werden.